# EUROPEAN PATENT APPLICATION

(11) **EP 4 586 639 A1**
(43) Date of publication of application: **16.07.2025**
(21) Application number: 23862772.3
(22) Date of filing: 03.07.2023
(51) Int. Cl.: H04R 1/46, A61B 7/04, H04R 19/04

(54) **BIOLOGICAL SOUND DETECTION DEVICE**

(30) Priority: 09.09.2022 JP 2022143775
(71) Applicant: Nisshinbo Micro Devices Inc., Tokyo 103-8456 (JP)
(72) Inventor: KUCHIJI, Hiroyuki, Fujimino-shi, Saitama 356-8510 (JP); MASUMOTO, Naoki, Fujimino-shi, Saitama 356-8510 (JP); SAKAI, Ryosuke, Fujimino-shi, Saitama 356-8510 (JP); FUJIWARA,Hajime, Fujimino-shi, Saitama 356-8510 (JP); SESHIMOTO, Akira, Fujimino-shi, Saitama 356-8510 (JP)
(74) Representative: M. Zardi & Co S.A.
(86) International application number: PCT/JP2023/024667
(87) International publication number: WO 2024/053227

(57) **Abstract**

Provided is a biological sound detection device that can detect a desired biological sound signal while suppressing a signal level of a noise signal. The biological sound detection device (100) comprises a sound collecting space (3) for a biological sound, a film (8) covering an opening thereof, a microphone (2) arranged in a rear chamber communicating with the sound collecting space (3), and a signal processing section (7) that signal processes the biological sound signal. The microphone (2) is constituted of a capacitive-type microphone and arranged so that a pressure applied to the film (8) passes through a backplate and is applied to a vibrating membrane and a displacement of the vibrating membrane caused by the pressure is initiated from a displacement in a direction away from the backplate. A volume of a space between the film (8) and the vibrating membrane is set so that the vibrating membrane does not come into contact with the backplate when the vibrating membrane displaces in a direction approaching the backplate and the displacement of the vibrating membrane becomes a displacement that can convert the biological sound into the biological sound signal.

## Description

### TECHNICAL FIELD

The present invention relates to a biological sound detection device with a microphone that detects a biological sound and converts it into a biological sound signal.

### BACKGROUND ART

A biological sound detection device, such as an electronic stethoscope, that converts a biological sound into a biological sound signal using a microphone, is widely used. The biological sound detection device is generally configured to perform signal processing such as amplification and filtering to remove noise on a biological sound signal converted from a biological sound and output the biological sound signal as a biological sound output signal. A configuration in which the biological sound output signal output from the biological sound detection device is output to an earpiece or the like, or output to an output device in a remote location via a communication line for a remote medical treatment, and the like are also known. Moreover, it is expected that a diagnostic accuracy will be improved by applying machine learning and statistical methods, including artificial intelligence, to the biological sound output signal.

For example, as an example of this type of biological sound detection device, an acoustic-electric transducer using a capacitive-type microphone is disclosed in Patent Document 1. Moreover, a capacitive-type microphone that can be used in this type of biological sound detection device is disclosed in Patent Document 2.

### PRIOR ART DOCUMENT

### Patent Document

Patent Document 1: JP 2003-527161 A
Patent Document 2: JP 2011-55087 A

### SUMMARY OF THE INVENTION

### PROBLEMS TO BE SOLVED BY THE INVENTION

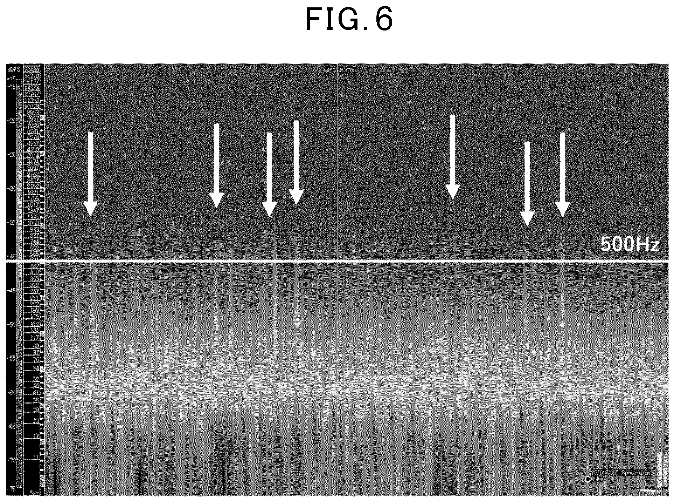

For example, in a biological sound detection device with a capacitive-type microphone as disclosed in Patent Document 1, when the biological sound detection device comes into contact with a body surface of a person to be auscultated, a capacitance value changes significantly and a large biological sound output signal is output. FIG. 6 is a spectrogram of heart sounds acquired using a biological sound detection device with a general capacitive-type microphone. In FIG. 6, the vertical axis indicates frequency, and the horizontal axis indicates time. In the spectrogram shown in FIG. 6, a biological sound output signal in a range of a frequency band exceeding 500 Hz can be confirmed. Generally, a frequency band of heart sounds or murmurs is 500 Hz or less. Moreover, a frequency band above 500 Hz includes a signal that is generated when the biological sound detection device comes into contact with the body surface of the person to be auscultated and a signal that is generated by vibration of a hand holding the biological sound detection device, and these signals become unnecessary noise signals when making a diagnosis. It can be seen that the spectrogram shown in FIG. 6 also includes such noise signals, as indicated by arrows, in addition to the signals due to heart sounds.

In order to suppress output of noise signals, for example, a biological sound detection device can be configured to be set to a state where the biological sound signal or the biological sound output signal is not output when the biological sound detection device comes into contact with the body surface of the person to be auscultated, and then switched to a state where the biological sound signal or the biological sound output signal is output. However, an operation of switching an output state is cumbersome, and touching the biological sound detection device when performing the switching operation may result in generation of new noise signals. Therefore, it is preferable to have a configuration in which noise signals are not output as much as possible without such an operation.

Therefore, it is an object of the present invention to provide a biological sound detection device that can detect a desired biological sound signal while suppressing a signal level of a noise signal generated when the biological sound detection device comes into contact with a body surface of a person to be auscultated.

### MEANS TO SOLVE THE PROBLEM

The biological sound detection device of the present invention comprises a sound collecting space for a biological sound, a film covering an opening of the sound collecting space, a microphone that is arranged in a rear chamber communicating with the sound collecting space and converts the biological sound into a biological sound signal, and a signal processing section for signal processing the biological sound signal, wherein the microphone is constituted of a capacitive-type microphone that generates a biological sound signal from a change in capacitance between a vibrating membrane that vibrates depending on a magnitude of a biological sound acquired in the sound collecting space and a backplate arranged opposite the vibrating membrane, and arranged so that a pressure applied to the film covering the opening of the sound collecting space passes through the backplate and is applied to the vibrating membrane and a displacement of the vibrating membrane caused by the pressure is initiated from a displacement in a direction away from the backplate, and wherein a volume of a space between the film covering the opening of the sound collecting space and the vibrating membrane is set so that the vibrating membrane does not come into contact with the backplate when the vibrating membrane displaces in a direction approaching the backplate and the displacement of the vibrating membrane becomes a displacement that can convert the biological sound into the biological sound signal.

### EFFECTS OF THE INVENTION

According to the present invention, the biological sound detection device is configured so that the vibrating membrane displaces in a direction away from the backplate when the biological sound detection device comes into contact with the body surface of the person to be auscultated, and therefore, a capacitance value detected at that time becomes very small, and generation of large noise signals can be suppressed. Furthermore, the volume of the space between the film covering the opening of the sound collecting space and the vibrating membrane is set to a volume that prevents contact between the vibrating membrane and the backplate and results in a displacement that can convert the biological sound into the biological sound signal, therefore making it possible to detect a biological sound signal at a desired signal level.

### BRIEF DESCRIPTION OF THE DRAWINGS

FIG. 1 is a schematic cross-sectional view of a biological sound detection device being one embodiment of the present invention (Embodiment 1).
FIG. 2 is an enlarged schematic cross-sectional view showing a portion II of the biological sound detection device of FIG. 1.
FIG. 3 is a graph illustrating a relationship between a volume of a space between a film covering an opening of a sound collecting space and a vibrating membrane and sensitivity at 100 Hz, in the biological sound detection device of Embodiment 1.
FIG. 4 is a spectrogram of heart sounds acquired using the biological sound detection device of Embodiment 1.
FIG. 5 is a schematic cross-sectional view, particularly of a microphone portion, of the biological sound detection device being another embodiment of the present invention (Embodiment 2).
FIG. 6 is a spectrogram of heart sounds acquired using a biological sound detection device with a capacitive-type microphone of a general related art.

### EMBODIMENT FOR CARRYING OUT THE INVENTION

The biological sound detection device of the present invention will be described with reference to the drawings. However, the present invention is not limited to these embodiments, and members, materials, and the like described below can be modified in various ways within the range of the gist of the present invention. In the drawings, the same reference numerals indicate the equivalent or same parts, and sizes and positional relationships between respective components are for convenience purposes and may not strictly reflect actual states.

### (Embodiment 1)

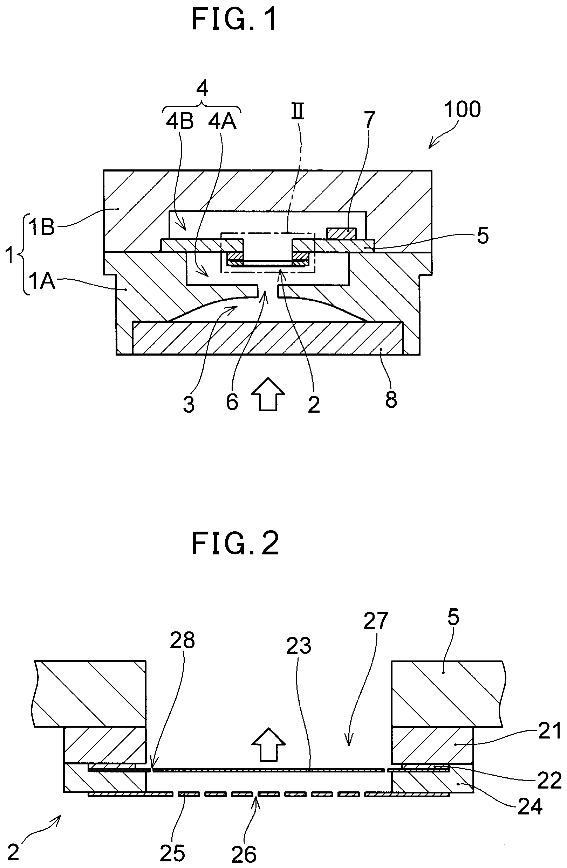

FIG. 1 is a schematic cross-sectional view illustrating Embodiment 1 of a biological sound detection device of the present invention, and FIG. 2 is an enlarged schematic cross-sectional view showing a portion II (a portion including a microphone) of the biological sound detection device of this embodiment shown in FIG. 1. As shown in FIG. 1, the biological sound detection device 100 of this embodiment has a microphone 2 arranged inside a housing 1 that consists of a base portion 1A and a lid portion 1B. The microphone 2 comprises a capacitive-type microphone.

The base portion 1A constituting the housing 1 is configured to have a sound collecting space 3 and a recessed portion 4A constituting a part of a rear chamber 4 that communicates with the sound collecting space 3. The microphone 2 is mounted on a substrate 5, which is placed on the base portion 1A so that the microphone 2 is arranged inside the recessed portion 4A. The lid portion 1B constituting the housing 1 is configured to have a recessed portion 4B constituting a part of the rear chamber 4 that communicates with the sound collecting space 3. A signal processing section 7 is mounted on the substrate 5, which performs processes such as amplification and filtering and generates an output signal, for a biological sound signal converted from a biological sound by the microphone 2. The lid portion 1B is arranged so that the signal processing section 7 is arranged inside the recessed portion 4B and a part of the substrate 5 is sandwiched between the lid portion 1B and the base portion 1A to be fixed.

The opening of the sound collecting space 3 is configured to be covered with a film 8. The film 8 is selected from materials suitable for contacting the body surface of the person to be auscultated to collect desired biological sounds. Specifically, for the film 8, a polyurethane gel (e.g., Echo Gel Pad manufactured by Yasojima Proceed Co., Ltd.), an urethane gel (e.g., Airlight Gel manufactured by Nisshinbo Chemical Inc.), a polyolefin-based thermoplastic resin (e.g., Cosmo Super Gel manufactured by COSMO **INSTRUMENTS** CO., LTD.), or the like can be used. Moreover, for the film 8, appropriate hardness and thickness for collecting desired biological sounds are selected. For example, when detecting heart sounds as biological sounds, the film 8 may be configured so that appropriate material and thickness are selected by comprehensively determining whether a low frequency band including heart sounds is stable, whether First Heart Sound and Second Heart Sound are clear, and whether there is no variation, etc. With the configuration being provided with the film 8, a variation in sensitivity can be suppressed.

The microphone 2 constituted of a capacitive-type microphone is joined to the substrate 5 as shown in FIG. 2. Specifically, a support substrate 21 composed of, for example, a silicon substrate or the like is joined to the substrate 5. An insulating film 22 composed of, for example, a thermal oxide film or the like is laminated on the support substrate 21, and a vibrating membrane 23 serving as a conductive movable electrode composed of, for example, polysilicon or the like is laminated on this insulating film 22. Furthermore, a backplate 25 including a conductive fixed electrode composed of, for example, polysilicon or the like and an insulating film composed of, for example, silicon nitride or the like is laminated on the vibrating membrane 23 via an insulating spacer 24 composed of, for example, a USG (Undoped Silicate Glass) film or the like. Reference numeral 26 denotes an acoustic hole formed in the backplate 25, Reference numeral 27 denotes a back chamber formed in the support substrate 21, and Reference numeral 28 denotes a slit formed in the vibrating membrane 23.

The biological sound detection device 100 shown in FIGS. 1 and 2 is configured to detect a biological sound and output a biological sound output signal as follows. First, when the biological sound detection device 100 comes into contact with a body surface of a person to be auscultated, a pressure is applied to the film 8 in the direction of the arrow shown in FIG. 1. This pressure causes the film 8 to deform so as to become convex on the sound collecting space 3 side, transmitting the pressure from the sound collecting space 3 to the rear chamber 4. The pressure applied to the film 8 is generated not only by biological sounds such as heart sounds, but also by pressing the biological sound detection device 100 against the body surface of the person to be auscultated due to contact between the biological sound detection device 100 and the body surface of the person to be auscultated or vibration of a hand touching the biological sound detection device 100. The pressure when the biological sound detection device 100 comes into contact with the body surface of the person to be auscultated and the pressure pressing the biological sound detection device 100 against the body surface of the person to be auscultated due to vibration of the hand holding the biological sound detection device 100 are much greater than the pressure generated by biological sounds.

The microphone 2 arranged inside the rear chamber 4 generates an electrical signal (a biological sound signal) depending on the pressure transmitted to the rear chamber 4. Here, in the microphone 2 of this embodiment, when a pressure is applied to the film 8 shown in FIG. 1 in the direction of the arrow, the vibrating membrane 23 shown in FIG. 2 is displaced in the direction away from the backplate 25 (the direction of the arrow) to initiate vibration. This displacement is a displacement that reduces an electrostatic capacitance value between the vibrating membrane 23 and the backplate 25 and does not generate a large noise signal. Then, the vibrating membrane 23 is displaced in a direction approaching the backplate 25 (an opposite direction to the arrow), but this displacement becomes smaller compared to the initial displacement in the direction away from the backplate 25. Moreover, as the noise signal is also generated by contact between the vibrating membrane 23 and the backplate 25, such generation of the noise signal can be suppressed by making a configuration that prevents this contact.

Therefore, in this embodiment, in order to make a configuration that prevents contact between the vibrating membrane 23 and the backplate 25, the volume of the space between the film 8 covering the opening of the sound collecting space 3 and the vibrating membrane 23 is adjusted. When the volume of the space between the film 8 and the vibrating membrane 23 (a sum of a volume of the sound collecting space 3, a volume of a through hole 6, a volume of a space surrounded by the recessed portion 4A of the rear chamber 4 and the substrate 5 and the microphone 2, and a volume between the vibrating membrane 23 of the microphone 2 and the backplate 25) is sufficiently larger compared with an amount of change in volume of the sound collecting space 3 caused by deformation of the film 8, the displacement of the vibrating membrane 23 is small, and the vibrating membrane 23 and the backplate 25 do not come into contact with each other. However, when the volume of the space between the film 8 and the vibrating membrane 23 is not significantly different from the amount of change in volume of the sound collecting space 3 caused by deformation of the film 8, the displacement of the vibrating membrane 23 becomes large, and the vibrating membrane 23 and the backplate 25 come into contact with each other, resulting in generation of a noise signal. That is, the volume of the space between the film 8 and the vibrating membrane 23 may be set to be sufficiently larger compared with the amount of change in volume of the sound collecting space 3 caused by deformation of the film 8.

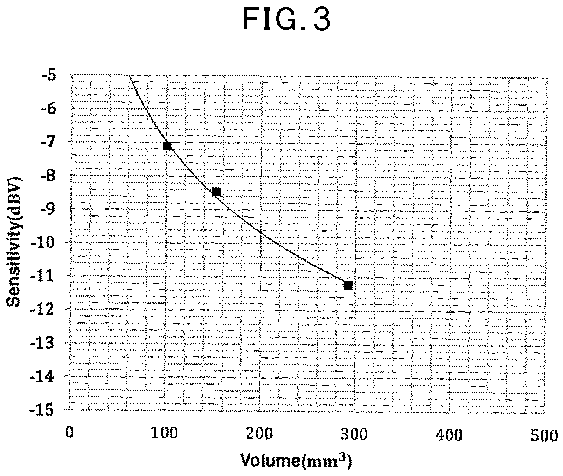

On the other hand, it is known that, if the volume of the space between the film 8 and the vibrating membrane 23 is made larger than necessary, sensitivity decreases. FIG. 3 is a graph illustrating a relationship between the volume of the space between the film 8 and the vibrating membrane 23 and the sensitivity. The example shown in FIG. 3 shows the relationship between the volume of the space between the film 8 and the vibrating membrane 23 and the sensitivity in a case of setting so as to generate a vibration of 100 Hz with a constant strength in the film 8 where an area of the opening of the sound collecting space 3 is 200 mm² and a load onto the biological sound detection device is 100 g using a polyurethane gel having a thickness of 4 mm as the film 8. Here, "sensitivity" indicates an output voltage from the biological sound detection device in logarithmic notation. As shown in FIG. 3, it can be seen that sensitivity decreases as the volume increases to 101 mm³, 153 mm³, and 293 mm³.

The relationship between the volume and sensitivity shown in FIG. 3 varies if the size of the sound collecting space 3, deformability of the film 8, a distance between the vibrating membrane 23 and the backplate 25, vibration characteristics of the vibrating membrane 23, and the like change. Thus, conditions such as the size of the sound collecting space 3 and the like may be appropriately set, the volume of the space between the film 8 and the vibrating membrane 23 may be increased so that, when the vibrating membrane 23 is displaced in a direction approaching the backplate 25, the vibrating membrane 23 vibrates without coming into contact with the backplate 25, and the volume may be set so that sensitivity is high (the biological sound can be converted into a biological sound signal of a desired signal level).

In this manner, the biological sound detection device 100 of this embodiment sets the volume of the space between the film 8 and the vibrating membrane 23 corresponding to deformability of the film 8. In this case, the biological sound detection device 100 needs to come into contact with a body surface of a person to be auscultated with a constant pressure. Therefore, if the biological sound detection device 100 is configured to come into contact with a body surface of a person to be auscultated by its own weight, it becomes possible to always make the contact with a constant pressure. For example, when the biological sound detection device 100 of this embodiment is placed on the chest of the person to be auscultated lying on his/her back, it comes into contact with the body surface of the person to be auscultated with a predetermined pressure, making it possible to detect heart sounds. In this case, it is possible to detect biological sounds without an auscultator or a person to be auscultated touching the biological sound detection device 100, so that noise signals generated by the auscultator or the like touching the biological sound detection device 100 are reduced. Moreover, such a configuration is appropriate for the biological sound detection device 100 used in a remote medical treatment in which a person to be auscultated who is unfamiliar with the operation must operate the detection device himself/herself.

The biological sound detected by the biological sound detection device 100 of this embodiment is converted into a biological sound signal by the microphone 2, and the biological sound signal is further subjected to signal processing by the signal processing section 7 and output as a biological sound output signal. A configuration can be made, in which the biological sound output signal is output to an output device such as an earpiece, an earphone, a headphone, a speaker, and the like which are not shown, and an auscultator can recognize it. For a remote medical treatment, a configuration can also be made, in which the biological sound output signal is transmitted to an output device in a remote location from a mobile phone, a smartphone, a tablet, etc. via a communication line and the auscultator can recognize it in the remote location.

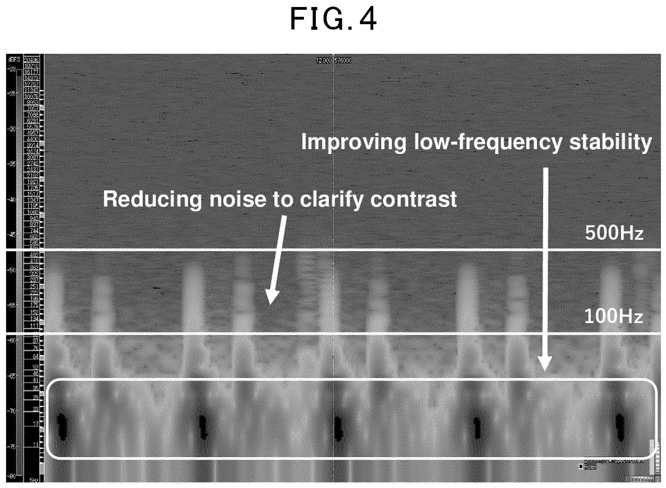

In addition, when the biological sound output signal is transmitted via a communication line, signals in some frequency bands may not be transmitted depending on a communication line used. For example, depending on a communication line, signal components at 100 Hz or less may be removed, and signal components around 200 Hz may be degraded. Therefore, the spectrogram shown in FIG. 4 can be used as the biological output signal. In this case, a configuration can be made, in which the spectrogram is generated by the signal processing section 7. Alternatively, the signal processing section 7 may generate a biological sound output signal to be transmitted to a spectrogram generating device, and the spectrogram generating device may generate a spectrogram upon receipt of the biological sound output signal. Image data like spectrogram are appropriate for performing artificial intelligence diagnosis. The biological sound output signal output from the biological sound detection device of this embodiment is particularly appropriate because noise signals are removed or reduced.

### (Embodiment 2)

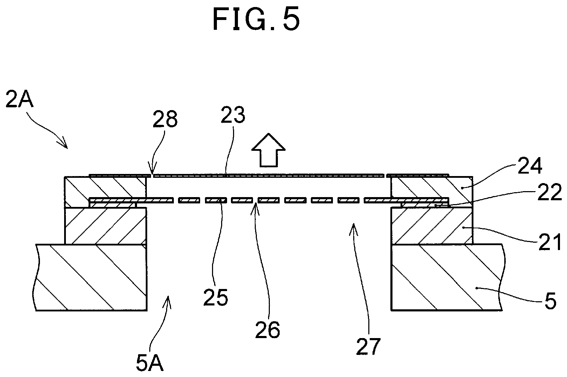

Next, Embodiment 2 of the biological sound detection device of the present invention will be described. FIG. 5 is a schematic partial cross-sectional view for explaining, particularly a microphone portion of, the biological sound detection device of this embodiment, and corresponds to FIG. 2 in the explanation of Embodiment 1. The microphone 2A of this embodiment also constituted of a capacitive-type microphone. As shown in FIG. 5, the microphone 2A is joined to the substrate 5. Specifically, a support substrate 21 composed of, for example, a silicon substrate or the like is joined onto the substrate 5. An insulating film 22 composed of, for example, a thermal oxide film or the like is laminated on the support substrate 21, and on this insulating film 22, a backplate 25 including a conductive fixed electrode composed of, for example, polysilicon or the like and an insulating film composed of, for example, silicon nitride or the like is laminated. Furthermore, on the backplate 25, a vibrating membrane 23 serving as a conductive movable electrode composed of, for example, polysilicon or the like is laminated via an insulating spacer 24 composed of, for example, a USG (Undoped Silicate Glass) film or the like. Reference numeral 26 denotes an acoustic hole formed in the backplate 25, Reference numeral 27 denotes a back chamber formed in the support substrate 21, and Reference numeral 28 denotes a slit formed in the vibrating membrane 23. In the microphone 2A of this embodiment, the backplate 25 is arranged on the support substrate 21 side, and the vibrating membrane 23 is arranged via the spacer 24.

The microphone 2A shown in FIG. 5 can be arranged in place of the microphone 2 arranged in the biological sound detection device 100 shown in FIG. 1. In this case, the microphone 2A mounted on the substrate 5 is arranged inside the recessed portion 4B of the lid portion 1B that constitutes the housing 1. The signal processing section 7 is also mounted on the substrate 5. The biological sound detection device of this embodiment can be configured in the same manner as the biological sound detection device 100 described in Embodiment 1, except that a structure of the microphone 2A differs from that of the microphone 2 and a mounting structure of the microphone 2A on the substrate 5 differs from that of the microphone 2 on the substrate 5.

In the biological sound detection device equipped with the microphone 2A, when a pressure is applied to the film 8 in the direction of the arrow as shown in FIG. 1, the vibrating membrane 23 of the microphone 2A is displaced in the direction away from the backplate 25 (the direction of the arrow) as shown in FIG. 5 to initiate vibration. This displacement is a displacement that reduces an electrostatic capacitance value between the vibrating membrane 23 and the backplate 25 and does not generate a large noise signal. Then, the vibrating membrane 23 is displaced in a direction approaching the backplate 25 (an opposite direction to the arrow), but this displacement becomes smaller compared to the initial displacement in the direction away from the backplate 25. Moreover, as a noise signal is generated by contact between the vibrating membrane 23 and the backplate 25, the generation of the noise signal can be suppressed by making a configuration that prevents this contact.

Therefore, also in this embodiment, in order to make a configuration that prevents contact between the vibrating membrane 23 and the backplate 25, the volume of the space between the film 8 covering the opening of the sound collecting space 3 and the vibrating membrane 23 is adjusted. In this embodiment, the volume of the space between the film 8 and the vibrating membrane 23 is a sum of a volume of the sound collecting space 3, a volume of the through hole 6, a volume of a space surrounded by the recessed portion 4A of the rear chamber 4 and the substrate 5, a volume of a through hole 5A in the substrate 5, a volume of a back chamber 27, and a volume of a space between the backplate 25 and the vibrating membrane 23. The volume of the space between the film 8 and the vibrating membrane 23 may be set to be sufficiently large compared with the amount of change in volume of the sound collecting space 3 caused by deformation of the film 8. Meanwhile, this volume is set to be a volume capable of converting a biological sound into a biological sound signal of a desired signal level, which is similarly applied in the biological sound detection device of this embodiment.

The microphone 2A shown in FIG. 5 can also be arranged inside the recessed portion 4A of the base portion 1A that constitutes the housing 1 shown in FIG. 1. Specifically, it may be arranged so that the substrate 5, to which the microphone 2A shown in FIG. 5 is joined, is joined onto the bottom of the recessed portion 4A. In this case, the volume of the space between the film 8 and the vibrating membrane 23 is a sum of a volume of the sound collecting space 3, a volume of the through hole 6, a volume of the through hole 5A in the substrate 5, a volume of the back chamber 27, and a volume of the space between the backplate 25 and the vibrating membrane 23. The volume of the space between the film 8 and the vibrating membrane 23 may be set to be sufficiently large compared with the amount of change in volume of the sound collecting space 3 caused by deformation of the film 8. This volume is also similarly set to be a volume capable of converting a biological sound into a biological sound signal of a desired signal level.

### (Conclusion)

(1) One embodiment of the biological sound detection device of the present invention is a biological sound detection device comprising a sound collecting space for a biological sound, a film covering an opening of the sound collecting space, a microphone that is arranged in a rear chamber communicating with the sound collecting space and converts the biological sound into a biological sound signal, and a signal processing section for signal processing the biological sound signal, wherein the microphone is constituted of a capacitive-type microphone that generates a biological sound signal from a change in capacitance between a vibrating membrane that vibrates depending on a magnitude of a biological sound acquired in the sound collecting space and a backplate arranged opposite the vibrating membrane, and arranged so that a pressure applied to the film covering the opening of the sound collecting space passes through the backplate and is applied to the vibrating membrane and a displacement of the vibrating membrane caused by the pressure is initiated from a displacement in a direction away from the backplate, and wherein a volume of a space between the film covering the opening of the sound collecting space and the vibrating membrane is set so that the vibrating membrane does not come into contact with the backplate when the vibrating membrane displaces in a direction approaching the backplate and the displacement of the vibrating membrane becomes a displacement that can convert the biological sound into the biological sound signal.

According to the biological sound detection device of the embodiment in (1) above, the vibrating membrane displaces in a direction away from the backplate when the biological sound detection device comes into contact with a body surface of a person to be auscultated, so that a capacitance value detected becomes very small, and generation of large noise signals can be suppressed. Furthermore, the volume of the space between the film covering the opening of the sound collecting space and the vibrating membrane is set so as to prevent contact between the vibrating membrane and the backplate and result in a displacement that can convert the biological sound into the biological sound signal, therefore making it possible to detect a biological sound signal at a desired signal level.

(2) The biological sound detection device in (1) above can be configured to have a weight that allows the biological sound to be acquired in the sound collecting space by its own weight when the biological sound detection device is brought into contact with a body surface of a person to be auscultated.

According to the biological sound detection device of the embodiment in (2) above, it is possible to detect biological sounds without an auscultator or a person to be auscultated touching the biological sound detection device, so that noise signals generated by the auscultator or the like touching the biological sound detection device are reduced. Moreover, such a configuration is appropriate for the biological sound detection device used in a remote medical treatment in which the person to be auscultated who is unfamiliar with the operation must operate the detection device himself/herself.

### REFERENCE SIGNS LIST

1. Housing
1A. Base portion
1B. Lid portion
2, 2A. Microphone
3. Sound collecting space
4. Rear chamber
4A, 4B. Recessed portion
5. Substrate
5A, 6. Through hole
7. Signal processing section
8. Film
21. Support substrate
22. Insulating film
23. Vibrating membrane
24. Spacer
25. Backplate
26. Acoustic hole
27. Back chamber
28. Slit

## Claims

1. A biological sound detection device comprising: a sound collecting space for a biological sound,
a film covering an opening of the sound collecting space;
a microphone that is arranged in a rear chamber communicating with the sound collecting space and converts the biological sound into a biological sound signal; and
a signal processing section for signal processing the biological sound signal;
wherein the microphone is
constituted of a capacitive-type microphone that generates a biological sound signal from a change in capacitance between a vibrating membrane that vibrates depending on a magnitude of a biological sound acquired in the sound collecting space and a backplate arranged opposite the vibrating membrane, and
arranged so that a pressure applied to the film covering the opening of the sound collecting space passes through the backplate and is applied to the vibrating membrane and a displacement of the vibrating membrane caused by the pressure is initiated from a displacement in a direction away from the backplate; and
wherein a volume of a space between the film covering the opening of the sound collecting space and the vibrating membrane is
set so that the vibrating membrane does not come into contact with the backplate when the vibrating membrane displaces in a direction approaching the backplate and the displacement of the vibrating membrane becomes a displacement that can convert the biological sound into the biological sound signal.

2. The biological sound detection device of claim 1, wherein the biological sound detection device has a weight that allows the biological sound to be acquired in the sound collecting space by its own weight when the biological sound detection device is brought into contact with a body surface of a person to be auscultated.
